# EUROPEAN PATENT APPLICATION

(11) **EP 4 321 500 A1**
(43) Date of publication of application: **14.02.2024**
(21) Application number: 22306194.6
(22) Date of filing: 08.08.2022
(51) Int. Cl.: C07C 67/03, C07C 67/52, C07C 51/09, C07C 51/43

(54) **METHOD AND INSTALLATION FOR THE PRODUCTION OF A MONOMER BY DEPOLYMERIZATION OF THE CORRESPONDING POLYMER**

(71) Applicant: ARKEMA FRANCE, 92700 Colombes (FR); Trinseo Europe GmbH, 8810 Horgen (CH)
(72) Inventor: DUBOIS, Jean-Luc, 92700 COLOMBES (FR); VAN DER HEIJDEN, Simon, 3402SE IJSSELSTEIN (NL)
(74) Representative: Lavoix

(57) **Abstract**

The invention relates to a method for the production of a monomer from a feed material containing a polymer of which the monomer is a constituent, the production method comprising the steps of:
- depolymerizing the feed material to obtain a monomer solution containing the monomer and impurities; and
- purifying the monomer solution thereby obtaining a purified monomer solution having a content of impurities lower than the content of impurities of the monomer solution,
wherein the purification step comprises at least one crystallization step each crystallization step comprising the steps of:
a) cooling of the monomer solution such that the monomer solution forms monomer crystals and a mother liquor,
b) separating the monomer crystals from the mother liquor, and
c) melting the monomer crystals thereby obtaining an enriched monomer solution.

## Description

### FIELD OF THE INVENTION

The project leading to this patent application has received funding from the European Union's Horizon 2020 research and innovation program under grant agreement No 820687.

The invention relates to the field of depolymerization for recovering a monomer from a feed material containing a polymer from which the monomer is a constituent.

In particular, the invention relates to the field of depolymerization of polymethyl methacrylate (PMMA) for recovering methyl methacrylate (MMA).

### BACKGROUND OF THE INVENTION

MMA is obtained for example by pyrolyzing PMMA in a pyrolysis reactor such as to obtain a gas stream including gaseous MMA and unavoidable impurities, separating solid impurities from the gas stream in a separator and condensing the gas stream to obtain liquid MMA. The MMA obtained from depolymerization of the PMMA is sometimes named crude MMA.

The liquid MMA obtained from depolymerization of PMMA may contain impurities, such as other chemical compounds present in the raw material containing the PMMA or generated during the depolymerization, e.g. during the pyrolysis.

The liquid MMA may be purified. Purification of the crude MMA may be operated by distillation, which is a process of the components from a liquid mixture by using selective evaporation and condensation, and separation based on the boiling point of the components or of their azeotropes.

### SUMMARY OF THE INVENTION

One of the aims of the invention is to propose a process of producing monomer from recycling of the polymer that allows obtaining a monomer solution with a satisfactory purity.

To this end, the invention propose a method for the production of a monomer from a feed material containing a polymer of which the monomer is a constituent, the production method comprising the steps of:
- depolymerizing the feed material to obtain a monomer solution containing the monomer and impurities; and
- purifying the monomer solution thereby obtaining a purified monomer solution having a content of impurities lower than the content of impurities of the monomer solution,
   the purification step comprising at least one crystallization step each crystallization step comprising the steps of:
   a) cooling of the monomer solution such that the monomer solution forms monomer crystals and a mother liquor,
   b) separating the monomer crystals from the mother liquor, and
   c) melting the monomer crystals thereby obtaining an enriched monomer solution.

According to other advantageous aspects of the invention, the production method comprises one or several of the following features, taken individually or according to any technically possible combination:
- the purification step comprises subjecting the enriched monomer solution resulting from one crystallization step to another crystallization step;
- the purification step comprises subjecting the mother liquor resulting from one crystallization step to another crystallization step;
- the purification step comprises a water removal step;
- the water removal step comprises:
   + the cooling of the monomer solution to a cooling temperature thereby transforming any water present in the monomer solution into ice without crystallizing the monomer, and
   + separating the ice from the monomer solution,
      whereby the at least one crystallization step is operated on the monomer solution after removal from the water;
- the cooling temperature is above the crystallization temperature of the monomer contained in the monomer solution;
- the difference between the cooling temperature and the melting point of the monomer contained in the liquid stream is higher than 4°C, preferably higher than 6°C and/or lower than 30°C, preferably lower than 20°C, mode preferably lower than 10°C;
- during the water removal step, the monomer solution is cooled at least in part by heat exchange with the purified monomer solution resulting from crystallization;
- the production method comprises adding at least one anti-icing additive in the monomer solution before implementing the at least one crystallization step such as to inhibit formation of ice during the implementation of the at least one crystallization step;
- the at least one anti-icing additive includes methanol;
- the depolymerization step comprises the pyrolysis of the feed material in a pyrolysis reactor thereby generating a gas stream and the condensation of the gas stream in a condenser thereby obtaining the monomer solution;
- the depolymerization step comprises a separation step including removing solid and/or liquid impurities from the gas stream in a separating unit before condensing the gas stream;
- the production method comprises a step of cleaning the gas stream between the separating unit and the condenser using a gas cleaning unit fluidly connected in series between the separating unit and the condenser, the gas cleaning unit comprising a transfer column containing internals, the gas cleaning unit being fed with a fraction of the monomer solution exiting the condenser such that the gas stream flows upwardly in the transfer column and the monomer solution flows downwardly in the transfer column, heavy contaminants contained in the gas stream condense in liquid state on the internals and flow back down by gravity towards the bottom of the transfer column and preferably back to the separating unit;
- the separating unit comprises an auxiliary separator and a main separator fluidly connected in series;
- the production method comprises a step of cleaning the monomer solution between the condenser and the purification unit by passing the monomer solution successively through an evaporator and a condenser thereby removing heavies from the monomer solution;
- the production method comprises a washing step including the addition of water to the monomer solution upstream from the crystallization unit;
- the polymer is polymethyl methacrylate (PMMA) and the monomer is methyl methacrylate (MMA); and
- the number of crystallization step is equal or lower than five, in particular equal or lower than three.

The subject-matter of the invention is also an installation for the production of a liquid monomer from a feed material containing a polymer of which the monomer is a constituent, the installation being configured for implementing a production method as above defined.

### BRIEF DESCRIPTION OF THE DRAWINGS

The invention and its advantages will be better understood upon reading the following description that is given solely by way of non-limiting example and given with reference to the appended drawings, in which:
- Figure 1 is schematic diagram illustrating an installation for the production of a monomer solution from a feed material containing a polymer from which the monomer is a constituent;
- Figure 2 is schematic diagram illustrating a purification unit of the installation of Figure 1; and
- Figure 3 is schematic diagram illustrating an alternative purification unit of the production installation of Figure 1.

### DETAILED DESCRIPTION OF PREFERRED EMBODIMENTS

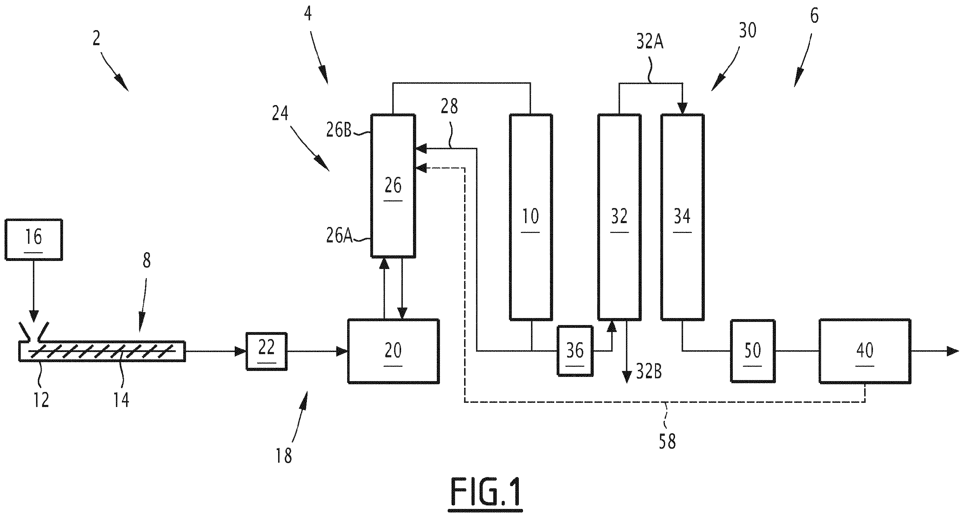

The installation 2 of Figure 1 is configured for the production of a monomer from a feed material containing a polymer of which the monomer is a constituent.

The installation 2 comprises a depolymerization unit 4 configured for depolymerizing the feed material to obtain a monomer solution containing the monomer and impurities, and a purification unit 6 configured for purifying the monomer solution such as to obtain a purified monomer solution having a content of impurities lower than the content of impurities of the monomer solution resulting from the depolymerization.

In the following, the monomer solution resulting from the depolymerization is also referred to as the "crude monomer solution" or "crude monomer".

The depolymerization unit 4 comprises for example a pyrolysis reactor 8 configured to receive the feed material and to generate a gas stream containing the monomer in gaseous state and a condenser 10 configured for condensing the gas stream such as to obtain the a monomer solution containing the monomer in liquid state.

The pyrolysis reactor 8 is for example a heated screw extruder comprising a heated tubular barrel 12 and at least one screw 14 arranged inside the barrel 12 for moving the feed material through the barrel 12 with heating the feed material.

The barrel 12 is preferably configured for heating the feed material according to a temperature profile, i.e. a temperature that varies along the barrel 12. The barrel 12 comprises for example a plurality or barrel sections which are configured for heating at different temperatures to obtain the temperature profile.

The depolymerization unit 4 comprises preferably a feeder 16 configured for feeding the feed material to an inlet of the pyrolysis reactor 8.

The depolymerization unit 4 comprises optionally a separation unit 18 connected in series between an outlet of the pyrolysis reactor 8 and the inlet of the condenser 10, the separation unit 18 being configured for separating solid impurities and/or liquid impurities from the gas stream before feeding the gas stream to the condenser 10.

The separation unit 18 comprises for example a main separator 20 configured for diminishing the linear velocity of the gas stream to promote the separation and deposit of solid impurities and/or for demisting the gas stream to promote the deposition of mist droplets of liquid impurities and/or for washing the gas stream with a washing liquid to promote deposition of liquid impurities.

The diminution of the linear velocity of the gas stream is obtained for example by a progressive increasing of the area of the cross-section of an internal flow passage of the main separator 20 through which the gas stream passes in the main separator 20 flows.

Preferably, the main separator 20 is configured such that the internal flow passage of the main separator 20 does not follow a straight line. Such a configuration promotes separation and deposition of impurities. The demisting of the gas stream is obtained e.g. by providing one or several demisting grids across the internal flow passage.

The washing of the gas is obtained e.g. by injection of water and/or injection of liquid monomer inside the flow passage.

The separation unit 18 comprises optionally an auxiliary separator 22 arranged upstream the main separator 20. The gas stream generated by the pyrolysis reactor 8 flow through the auxiliary separator 22 before entering the main separator 22.

The auxiliary separator 22 is for example configured for removing solid impurities from the hot gas stream, e.g. by inertia. The auxiliary separator 22 is for example configured with an internal flow passage having a change of cross section promoting a decrease of linear velocity and/or a change of direction that are located above a collecting pot such that the solid particles slowed down by the decrease of linear velocity and/or driven by inertia tend to hit walls of the internal flow passage and to fall down in the collecting pot.

Advantageously, the depolymerization unit 4 comprises a gas cleaning unit 24 connected in series between the separation unit 18 and the condenser 10 and configured for removing heavies from the gas stream before the gas stream enters the condenser 10.

In operation, the gas stream exiting the separation unit 18 passes via the gas cleaning unit 24 before entering the condenser 10.

The gas cleaning unit 24 comprises a transfer column 26 extending upwardly between a lower end 26A fluidly connected to the separation unit 18, in particular to the main separator 20, for receiving the gas stream exiting the separation unit 18 and an upper end 26B fluidly connected to an inlet of the condenser 10.

The gas cleaning unit 24 comprises a reinjection loop 28 configured for reinjecting a fraction of the monomer solution collected downstream the condenser 10 into a top portion of the transfer column 26, in particular at the top end 26B of the transfer column 26.

The transfer column 26 is provided with internals. The internals comprise for example baffles, structure packing, gauze packing and/or random packing, e.g. random packing including saddles and/or rings, in particular Pall rings.

The internals are configured such that, in operation, the gas stream flows upwardly in the transfer column, the temperature of the gas stream diminishing along the transfer column, and the monomer solution flows downwardly inside the transfer column 26 by gravity, heavy contaminants contained in the gas stream condense on the internals and flow back in liquid state down the transfer column 26 and into the separation unit 18, in particular into the main separator 20.

Preferably, the temperature of the gas stream at the outlet of the main separator 20 and entrance of the transfer column 26 is maintained at least at the boiling point of the monomer +20°C, in particular at least at the boiling point of the monomer +30°C, more in particular at least at the boiling point of the monomer +40°C, even more in particular at least at the boiling point of the monomer +50°C.

Preferably the gas stream at the outlet of the transfer column 26 is maintained between the boiling point of the monomer and the boiling point of the monomer +50°C, in particular between the boiling point of the monomer and the boiling point of the monomer +40°C, in particular between the boiling point of the monomer and the boiling point of the monomer +30°C, in particular between the boiling point of the monomer and the boiling point of the monomer +20°C, in particular between the boiling point of the monomer and the boiling point of the monomer +10°C.

Optionally, the purification unit 6 comprises a solution cleaning unit 30 configured for removing liquid impurities from the monomer solution provided by the depolymerization unit.

A solution cleaning unit 30 comprises for example an evaporator 32 and a condenser 34 fluidly connected in series for successively evaporating and condensing the monomer solution.

Upon evaporating and condensing the stream, it is possible to separate heavies of the monomer solution from the monomer and lights contained in the monomer solution.

The monomer and lights are for example provided at lights outlet 32A of the evaporator 32 that is connected to an inlet of the condenser 34 of the additional cleaning unit 30 while the heavies are evacuated at a heavies outlet 32B of the evaporator 32.

Alternatively or optionally, the solution cleaning unit 30 comprises one or several distillation columns fluidly connected in series, e.g. one or two distillation columns fluidly connected in series. Each distillation column allows separating the monomer from other compounds present in the monomer solution at this location of the installation 2.

Alternatively or optionally, the solution cleaning unit 30 comprises a thin-film evaporator or a short-path distillation unit or any other appropriate technology or combination of technologies.

Optionally, the purification unit 6 comprises a washing unit 36 configured for adding water and possibly other chemicals into the monomer solution provided by the depolymerization unit 4.

When a washing unit 36 and a solution cleaning unit 30 are provided in the purification unit 6, the washing unit 36 is preferably arranged upstream the solution cleaning unit 30.

The washing unit 36 is for example configured for addition of water into the monomer solution in one, two or more steps. In a first step, an alkaline aqueous solution is mixed with the monomer solution in order to trap organic and inorganic acids such as hydrochloric acid (HCI), methacrylic acid, acetic acid... After a decantation or centrifugation step, or any other suitable separation step, the aqueous and organic phases are separated. The organic phase that could be contaminated with remaining traces of alkaline compound is washed with fresh water. After a new decantation or other separation step, the organic phase can be directed to the next stage while the aqueous phase, that contains traces of alkaline compound and other organics partially soluble in water, is directed to the first alkaline washing step, not only to save water but also to recover some monomer partially soluble in water. The washing steps can be extended with one or more additional washing steps with other chemicals, in order to trap additional impurities. These additional washing steps can be conducted at any position in the washing sequence, but would preferably occur before the final clean water washing step.

After the washing steps, some of the remaining water remain soluble in the monomer solution.

The purification unit 6 comprises a crystallization unit 40 which is configured for performing at least one crystallization step such as to separate impurities from the monomer solution, each crystallization step comprising cooling the monomer solution such as to form crystals of the monomer and a mother liquor, separating the monomer crystals from the mother liquor and melting the monomer crystals thereby obtaining a monomer-enriched solution.

Such a crystallization allows removing from the monomer solution components that have a melting temperature that is strictly inferior to the melting temperature of the monomer. Such components are contained in the mother liquor.

A crystallization with removal of some components from the initial solution subjected to crystallization is also referred to as "fractional crystallization".

It is possible to subject the monomer solution to several crystallization steps in view of obtaining a monomer solution which has a high degree of purity. It is also possible to subject a mother liquor to a crystallization step, e.g. in view of recovering monomer still present in the mother liquor.

In one example in which the crystallization unit 40 is configured for performing several crystallization step, the crystallization unit 40 is configured for subjecting the enriched monomer solution resulting from a crystallization step to another crystallization step and/or for subjecting the mother liquor resulting from a crystallization step to another crystallization step.

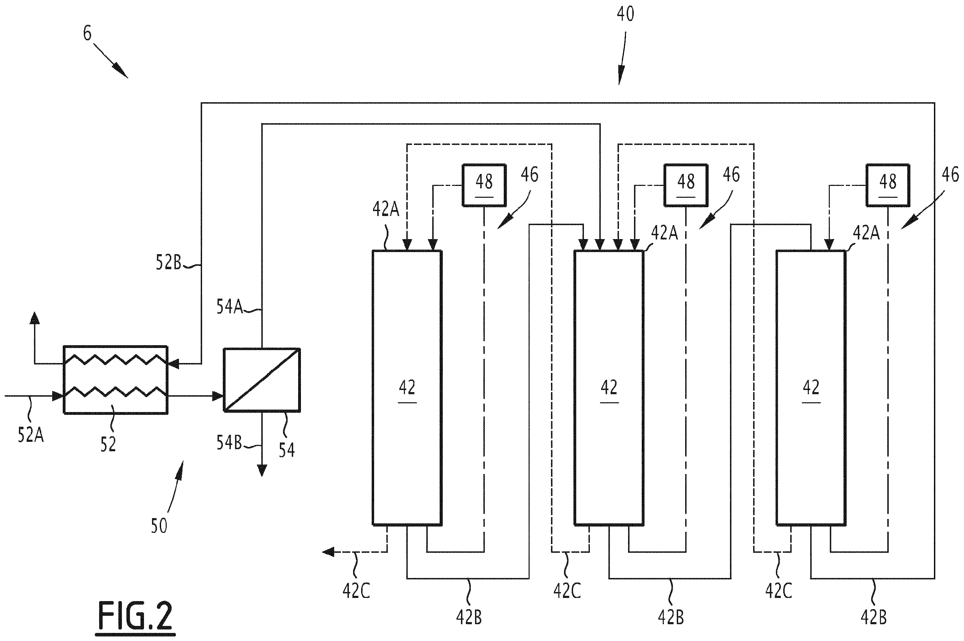

As illustrated on Figure 2, the crystallization unit 40 comprises one or several crystallizers 42, each crystallizer 42 being configured for performing a respective crystallization step. Each crystallizer 42 is configured for receiving liquid (either the monomer solution provided by the depolymerization unit 4 or the monomer-enriched solution provided by a preceding crystallizer 42), for cooling the liquid to crystallize the monomer to obtain a monomer-enriched solution and a mother liquor.

Each crystallizers 42 is for example configured for collecting the mother liquor and for collecting the monomer crystals as a monomer-enriched solution.

Each crystallizers 42 is for example configured for receiving the monomer solution at an inlet 42A for providing enriched monomer solution at a first outlet 42B and for providing the mother liquor at a second outlet 42C.

Each crystallizer 42 is for example configured for batch operation, i.e. for sequentially receiving a batch of monomer solution, subjecting the monomer solution to crystallization and collecting the mother liquor and the purified monomer before receiving a new batch of monomer solution.

Alternatively, each crystallizer 42 is configured for a semi-continuous operation, i.e. for a continuous flow of mother liquor through the crystallizer 42 and a recovery of the purified monomer at intervals, in particular regular intervals. Multi-tubular crystallizers are for example operable in a semi-continuous manner.

The crystallization unit 40 comprises advantageously a series of crystallizers 42 comprising a plurality of crystallizers 42 fluidly connected in series such that the monomer-enriched solution produced by each crystallizer 42 of the series of crystallizers 42 is furnished to the following crystallizer 42 and the mother liquor produced by each crystallizer 42 is provided to the preceding crystallizer 42 of the series of crystallizers 42.

The continuous flow of mother liquor from each following crystallizer 42 to each preceding crystallizer 42 makes it possible to implement a continuous operation. In addition, when the crystallization is conducted with multi-tubular crystallizers, it becomes possible to operate in a semi continuous process.

The crystallization unit 40 comprises for example two, three or more crystallizers 42 fluidly connected in series.

The crystallization unit 40 comprising a series of crystallizer 42 is also referred to as a "cascaded crystallization unit 40". It allows to perform a cascaded fractional crystallization.

The crystallization unit 40 comprising a series of crystallizers 42 comprises a first crystallizer 42 and a last crystallizer 42, and, optionally, at least one intermediate crystallizer 42 connected in series between the first crystallizer 42 and the last crystallizer 42.

The crystallization unit 40 is for example configured such that the monomer solution provided by the depolymerization unit 4 is fed to one of the series of crystallizers 42, preferably an intermediate crystallizer 42 of the series of crystallizers 42 when the crystallization unit 40 comprises three or more crystallizers 42 fluidly connected in series.

As illustrated on Figure 2, the crystallization unit 40 comprises three crystallizers 42 fluidly connected in series, including a first crystallizer 42 (on the left in Figure 2), an intermediate crystallizer 42 (in the middle in Figure 2) and a last crystallizer 42 (on the right on Figure 2).

The crystallization unit 40 is configured for providing the monomer solution produced by the depolymerization unit 4 to the inlet 42A of the intermediate crystallizers 42 via a feeding line.

The first outlet 42B of the intermediate crystallizers 42 is fluidly connected to the inlet of the last crystallizer 42. The second outlet 42C of the intermediate crystallizer 42 is fluidly connected to the inlet of the first crystallizer 42.

The first outlet 42B of the first crystallizer 42 is fluidly connected to the inlet 42A of the intermediate crystallizer 42. The second outlet 42C of the first crystallizer 42 provides the mother liquor stream.

The second outlet 42C of the last crystallizer 42 is fluidly connected to the inlet 42A of the intermediate crystallizer 42. The first outlet 42B of the last crystallizer 42 provide the processed liquid monomer.

The crystallization unit 40 comprises a respective crystal washing device 46 associated to each crystallizer 42, the crystal washing device 46 comprising a tank 48 configured for storing a washing monomer solution and for feeding the washing monomer solution to the crystallizer 42 for cleaning the monomer crystals contained in the crystallizer 42.

The crystallization unit 40 is configured such that the washing monomer solution provided to the tank 48 of each crystal washing device 46 is made of melted monomer crystals containing fewer impurities than the initial monomer solution provided to the crystallizer 42.

During the implementation of a crystallization step in each crystallizer 42, the crystals accumulate some impurities which might be trapped in the voids between the crystals or on the crystal surface. Before completely melting the crystals generated during the crystallization step, the washing device 46 is operable to feed the crystallizer 42 for washing the monomer crystals with the washing monomer solution. The impurity enriched fraction is then directed together with the mother solution to the previous crystallizer 42 or stored to be used in the next crystallization step operated in the same crystallizer 42. The monomer crystals are then melted completely and evacuated as a monomer-enriched solution.

As illustrated on Figure 2, the crystallization unit 40 is configured such that a fraction of the monomer-enriched solution produced by each crystallizer 42 is used as washing monomer solution for the same crystallizer 42.

To this end, the tank 48 of each washing device 46 associated to a crystallizer 42 and fluidly connected to this crystallizer 42 for collecting a fraction of the enriched monomer solution produced by this crystallizer as a washing monomer solution and for feeding the washing monomer solution to the crystallizer 42. Each tank 48 is fluidly connected in closed loop to the associated crystallizer 42.

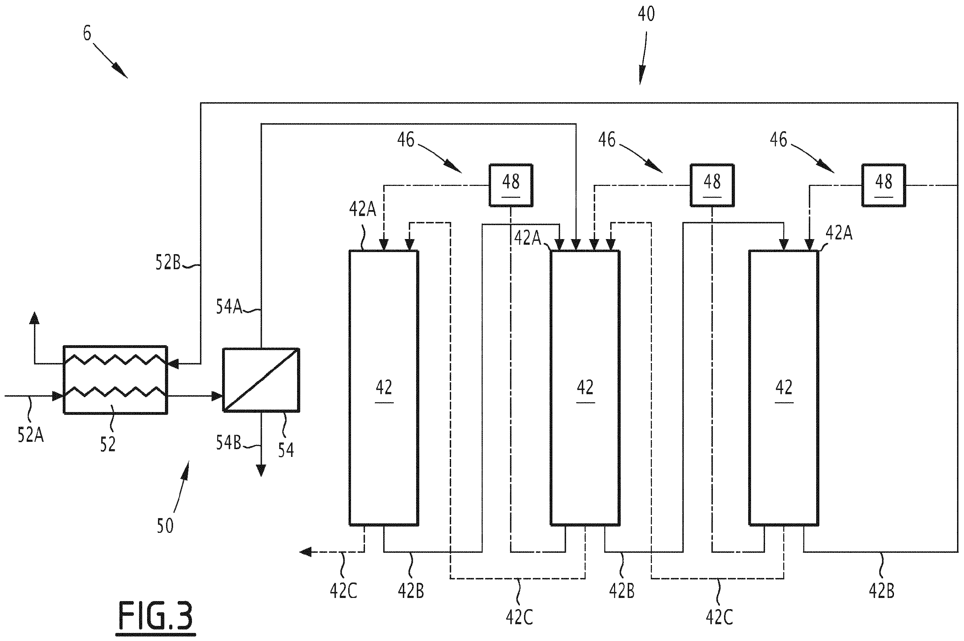

The crystallization unit 40 of Figure 3 differs from that of Figure 2 in that it is configured such that the monomer-enriched solution produced by each following crystallizer 42 is used as washing monomer solution for the preceding crystallizer 42. Preferably, the monomer-enriched solution produced by the last crystallizer 42 is used as washing monomer solution for the last crystallizer 42.

To this end, the tank 48 of the washing device 46 associated to each preceding crystallizer 42 is fed with a fraction of the monomer-enriched solution produced by the following crystallizer 42. In other words. The monomer-enriched solution produced by each following crystallizer 42 is used as washing monomer solution for the preceding crystallizer 42. The last crystallizer 42 is for example fed with washing monomer solution from a tank 48 that is fluidly connected to the first outlet 42B of the last crystallizer 42 providing the enriched monomer solution.

Preferably, the purification unit 6 comprises a water removal unit 50 arranged upstream the crystallization unit 40 for removing water from the crude monomer solution provided by the depolymerization unit 4 before feeding the monomer solution to the crystallization unit 40.

The water removal unit 50 comprises for example a cooling device 52 configured for cooling the monomer solution such as to crystallize water contained in the monomer solution into ice without crystallizing the monomer contained in the monomer solution and a removal device 54 configured for removing the ice from the monomer solution that is then fed to the crystallization unit 40.

The cooling device 52 is for example configured for cooling the monomer solution by heat exchange with another fluid. The cooling device 52 is a heat exchanger, in particular a liquid/liquid heat exchanger or a suspension crystallizer.

As illustrated in Figure 2, the other liquid is preferably the purified monomer solution produced by the crystallization unit 40. The outlet of the crystallization unit 40 providing the purified monomer solution is fluidly connected to the cooling device 52 which is configured for heat exchange between the monomer solution provided by the depolymerization unit 4 and the purified monomer solution provided by the crystallization unit 40.

The monomer solution provided by the depolymerization unit 4 is received at a first inlet 52A of the cooling device 52 and the purified monomer solution provided by the crystallization unit 40 is received at a second inlet 52B of the cooling device 52. The first inlet 52A and the second inlet 52B are not fluidly connected.

As a matter of fact, the purified monomer solution exiting the crystallization unit 40 exhibits a low temperature, in particular a temperature close the melting temperature of the monomer, and can be used for crystallization of the water contained in the monomer solution provided by the depolymerization unit 4, when the melting temperature of the monomer being strictly inferior to the crystallization temperature of water.

The removal device 54 is for example configured for removing the ice by filtration, accumulation on a cold surface, floatation, overflow, decantation and/or centrifugation.

The removal device 54 receives the monomer solution cooled by the cooling device 52 and provides the monomer solution at a first outlet 54A fluidly connected to the crystallization unit 40 and ice at a second outlet 54B.

The installation 2 of Figure 1 is operable to implement a method for the production of a monomer from a feed material containing a polymer of which the monomer is a constituent.

The production method comprises a step of depolymerizing the feed material to obtain a crude monomer solution containing the monomer and impurities, and a step of purifying the crude monomer solution, such as to obtain a purified monomer solution having a content of impurities lower than the content of impurities of the crude monomer solution resulting from the depolymerization.

The depolymerization step is performed in the depolymerization unit 4 and the purification step is performed in the purification unit 6.

The purification step comprises at least one crystallization step performed in the crystallization unit 40 such as to separate impurities from the monomer solution, each crystallization step comprising cooling the monomer solution such as to form crystals of the monomer and a mother liquor, separating the crystals from the mother liquor and melting the monomer crystals thereby obtaining a monomer-enriched solution. Each crystallization step is preferably performed in a respective crystallizer 42.

The purification step comprises subjecting the monomer-enriched solution resulting from a crystallization step to another crystallization step and/or subjecting the mother liquor resulting from a crystallization step to another crystallization step.

This is performed in the crystallization unit 40 comprising a series of crystallizers 42 respectively by feeding the monomer-enriched solution provided by a crystallizer 42 to the following crystallizer 42 and/or by feeding the mother liquor provided by a crystallizer 42 to the preceding crystallizer 42.

The crystallization steps performed in the crystallizers 42 are preferably performed at different temperatures, the crystallization temperature of the crystallization stage performed in each preceding crystallizer 42 being preferably strictly inferior to the crystallization temperature of the crystallization step performed in the following crystallizer 42.

Indeed, the purity of the monomer solution increases from each preceding crystallizer 42 to the following crystallizer 42 and it is thus possible to use a temperature progressively closer to the melting temperature of the monomer for progressively separating the monomer from liquid impurities having melting temperatures closer to that of the monomer.

The crystallization step advantageously comprises a water removal step performed in the water removal unit 50.

The water removal step includes cooling the crude monomer solution resulting from the depolymerization step to a cooling temperature such as to transform water present in the crude monomer solution into ice without crystallizing the monomer, separating the ice from the monomer solution, the crystallization step being operated on the monomer solution after removal from the water.

During the water removal step, the liquid stream is cooled at least in part by heat exchange with purified monomer solution provided by the crystallization unit 40.

The water removal step is performed in the purification unit 6 by the water removal unit 50, in particular the water removal unit 50 comprising the cooling device 52 and the removing device 54.

The cooling temperature is below the crystallization temperature of water and above the crystallization temperature of the monomer.

The difference between the cooling temperature and the melting point of the monomer contained in the liquid stream is higher than 4°C, preferably higher than 6°C and/or lower than 30°C, preferably lower than 20°C, mode preferably lower than 10°C.

The depolymerizing step comprises the pyrolysis of the feed material in the pyrolysis reactor 8 such as to generate a gas stream and the condensation of the gas stream in the condenser 10 such as to obtain the crude monomer solution.

The depolymerizing step advantageously comprises a separation step including removing solid and/or liquid impurities from the gas stream in the separating unit 18 before condensing the gas stream in the condenser 10.

The depolymerizing step advantageously comprises a step of cleaning the gas stream between the separating unit 18 and the condenser 10 using a gas cleaning unit 24 fluidly connected in series between the separating unit 18 and the condenser 10, the gas cleaning unit 24 comprising a transfer column 26 containing internals, the gas cleaning unit being fed with monomer solution such that the gas stream flows upwardly in the transfer column 26 and the monomer solution flows downwardly in the transfer column 26 and preferably back to the separating unit 18.

The purification step advantageously comprises a step of cleaning the monomer solution, with passing the stream though an optional solution cleaning unit 30.

The solution cleaning unit 30 is for example configured for passing the monomer solution through an evaporator 32 and then though a condenser 34 to condense with separating heavies form the monomer and lights.

The solution cleaning unit 30 comprises optionally or alternatively a distillation unit or a thin-film evaporator or a short-path distillation unit or any other appropriate technology or combination of technologies.

The optional solution cleaning unit 30 allows removing heavy compounds (or "heavies"). The lights compounds remaining in the monomer solution provided to the purification unit 6 are thus more likely to contain impurities which have a lower melting point than the monomer and which can be separated from the monomer by crystallization.

The purification step advantageously comprises a washing step including adding water in the monomer solution upstream from the crystallization unit 6 in a washing unit 36.

In one example, the polymer is polymethyl methacrylate (PMMA) and the monomer is methyl methacrylate (MMA).

The melting point of pure water is 0°C. The melting point of pure MMA is -48°C. Melting points are indicated here for a pressure of 1013 mbar.

It has been found that in practice, key impurities contained in crude MMA from depolymerization have a melting point strictly inferior to that of MMA and that can be removed efficiently with the crystallization step.

The impurities potentially present and their effective or predicted melting points are listed in the table below:

**Table 1: melting points of impurities**

| **Compound** | **Melting point** |
|---|---|
| Methacrylic Acid | +15°C |
| Acrylic Acid | +14°C |
| Benzene | +5.5°C |
| Water | 0°C |
| 2,3-butanedione | -2.4°C |
| Methyl-2-Hydroxylsobutyrate | -24.5°C |
| Methacrylonitrile | -35.8 °C |
| 2,2 dimethoxypropane | -47 °C |
| MMA | -48 °C |
| Dimethyl tetramethyl succinate, taken as dimer proxy | -57.84 °C (predicted) |
| Butyl Acrylate | -64°C |
| 2,2,4 trimethyl pentanedioate dimethyl ester, taken as dimer proxy | -64.2 °C |
| Vinyl Methacrylat | -68.4 °C (predicted) |
| 2-methyl-Methyl Butanoate | -68.4 °C |
| Ethyl Acrylate | - 71 °C |
| Ethyl Propionate | -73.6 °C |
| Methyl Acrylate | -74 °C |
| Ethyl Methacrylate | -75 °C |
| Buthyl Methacrylate | -75 °C |
| Methyl Pivalate | <-70°C |
| Dimethyl-2-methyl Glutarate, taken as dimer proxy | -82.7 °C (predicted) |
| Acrylonitrile | -84 °C |
| Methyl isobutyrate | -85 °C |
| Methyl Propionate | -88 °C |
| Butanol | -89.8 °C |
| MMA-Methyl Isobutyrate Eutectic | -92 °C |
| Toluene | -95°C |
| Methanol | -97.6 °C |

In an alternative example, instead of removing water from the liquid stream provided by the depolymerization unit 4, the production method comprises a step of adding at least one anti-icing additive in the monomer solution before implementing the crystallization step such as to inhibit formation of ice during the implementation of the crystallization step.

The at least one anti-icing additive includes for example methanol.

The inhibition of water crystals (ice) formation by addition of at least one anti-icing additive in the crude monomer solution allows to perform the crystallization step while removing key impurities even though some water is embedded in the monomer crystals and thus present in the monomer solution.

In particular, unexpectedly, when methanol is added to crude MMA solution with a ratio of methanol/MMA preferably above 4 g/100 g and/or preferably below 50 g/100 g, ice formation is inhibited and MMA crystals can be produced directly. The MMA crystals formed during crystallization are embedded with some water, but key impurities such as methyl isobutyrate, ethyl acrylate and toluene can be removed effectively.

Optionally, the purified MMA solution can still be purified by a distillation step operated after the crystallization step.

Molecules other than methanol could be used as anti-icing additive in the crude MMA, but methanol is already an impurity present in crude MMA and is rather inexpensive.

The invention is not limited to the examples described above and illustrated on Figures 1 - 3. Other embodiments can be contemplated.

For example, instead of having a series of crystallizers, the crystallization unit 40 may comprise only one single crystallizer 42.

As illustrated in Figure 1, optionally, at least a fraction of the mother liquor produced in the crystallization unit 40 is returned the gas washing unit 24 and in particular injected in a top portion 26B of the transfer column 26. In such case, a return line 58 is provided between the crystallization unit 40 and the transfer column 26. This allows recovering some monomer still present in the mother liquor.

Optionally, at least a fraction of the mother liquor produced by the crystallization unit 40 is used as a cold source in one or several heat exchangers.

### Examples

For the realization of examples, two solutions of water-ethanol were prepared with two different concentrations of ethanol. The two solutions were introduced in two distinct Dewar vessels. Dry ice was crushed into powdery material and slowly added in the Dewar vessels. When the solutions were bubbling more gently, larger pieces of dry ice were added in the Dewar vessels until the temperature stabilized and the solution took the consistency of a syrup. The first bath solution was adjusted (i.e. ethanol / water ratio was adjusted) to have a temperature comprised between -40°C and -42°C, while the second bath solution was adjusted to reach a temperature between -52°C and -57°C. By tuning the ethanol content in an aqueous solution, it is possible to tune the temperature at which the syrup will be formed and the temperature at which the temperature will stabilize.

Then two glass vessels of about 4 cm diameter and 30 cm length were introduced in the two Dewars vessels. About 100 ml of crude MMA was introduced in the first glass vessel, which reached about -40°C after about 30 minutes while adding dry ice in the Dewars vessels to stabilize the temperature. Some crystals formed in suspension and were filtered and the solid was recovered and melted only above 0 °C or when reaching room temperature. The filtration had to be done very rapidly to avoid that the solids would melt. A filter paper with low heat conductivity was used for this purpose. The collected solids were transferred in a sample vial. While doing the filtration, the liquid fraction was poured in the second glass vessel, which was at much lower temperature. Then, again the temperature of the bath was adjusted to reach less than -52°C, within about 20-30 minutes. After that time, about half of the liquid had solidified. A glass bar was introduced in the vessel while cooling and it was possible to see crystals forming on the glass bar. After that time, the liquid fraction was collected and poured in a collection flask. The solid was allowed to sweat for about 1-2 minutes and the liquid fraction which formed was added to the previous one. Then the rest of the solid was melted and was poured in a separate collection flask.

In some experiments, a slightly bigger amount of product was crystallized, and the solid that crystallized was melted and crystallized again to improve the purity of the solid fraction. In this case, two mother liquors liquid fractions were collected as well as one final crystallized fraction.

**Table 2: Crystallization conditions**

| Ex. | Crude MMA origin / PMMA type | 1^{st} crystallization | 2^{nd} crystallization | 3rd crystallization |
|---|---|---|---|---|
| 1 | (old crude) | -40 °C | -52 °C | -57 °C |
| 2 | old crude + 4 wt % Methanol | None at -40 °C | -57 °C | |
| 3 | Cast clear | -40 °C | -52 °C | |
| 4 | White cast (with TiO2 as pigment) | -42 °C | -52 °C | |
| 5 | 80 wt % Cast - 20 wt % Extrusion mixed colors and suppliers, representative of European mixed scraps from sheets. | -40 °C | -52 °C | |
| 6 | 80 wt % Cast - 20 wt % Extrusion mixed colors and suppliers, representative of European mixed scraps from sheets. | -40 °C | -52 °C | |
| 7 | Injection PMMA scraps from car tail lights containing red, black and transparent particles | -42 °C | -52 °C | |
| 8 | Mix of scraps white Cast PMMA used to make bath tubs, and coated on one side with glass fibers to reinforce the tub, with equal amount of impact resistant PMMA sheet | -42 °C | -52 °C | -52 °C |

### Example 1:

A crude MMA was obtained from PMMA depolymerized at 470 °C in a twin-screw extruder and produced while connecting directly the main separator 20 to the condenser 10. This means that the cleaning unit 26 and the main separator 22 were also omitted.

The product was initially cooled to -40 °C, to separate a first crystal fraction, and then to -52 °C, where the crystallized fraction and the mother liquor where collected. The crystallized fraction was collected, melted and crystallized again but at -57 °C, generating a second mother liquor and a new crystallized fraction. The initial crude MMA is orange. There is a small change in color of the final crystallized fraction.

### Example 2:

The previous example was repeated, but with adding 4 wt % of methanol in the crude MMA. More particularly, a first shot of 2 wt % Methanol was added and the solution was cooled to -40 °C whereby crystals were forming. So the solution was melted at room temperature, and a second shot of 2 wt % of methanol for a total of 4 wt %. Then the solution was cooled to -40 °C. No solid formation was observed. Then the liquid was further cooled to -57 °C, and left at this temperature for about 20 minutes. The liquid faction was collected, the sweated fraction was added to it and the solids melted and were collected separately. The initial crude MMA is orange. There is a small change in color of the final crystallized fraction.

### Comparative example 1

A sample of a transparent clear virgin MMA purchased from ALDRICH was used as a reference.

Then 100 mg of peroxide LUPEROX^{®} LP (dilauroyl peroxide) was added in about 5 ml of the liquid monomer solution in a test tube equipped with a polymer cap. The addition was done at room temperature, and the sample was shaken and then placed in a sample holder, which was in a tank of water kept at constant temperature of 65 °C with water circulation. Organic peroxide should be handled with care as they can decompose violently.

Within 2 minutes the color of the solution did not change. The sample was kept for two days, and even after that long period not difference in color could be detected.

### Example 3:

The crude MMA obtained from Cast Clear PMMA sheets was used in this test. The PMMA was depolymerized at 450 °C and then the temperature was increased to 470 °C in the twin-screw extruder, while the feed rate was increased from 50 to 90 kg/h. The hot vapor were collected, washed with cold crude MMA in counter current, and condensed in a condenser provided as a tube and shell heat exchanger (corresponding to condenser 10 of the installation of Figure 1).

A sample of a transparent clear solution was taken from the condenser.

Then 100 mg of peroxide LUPEROX^{®} LP (dilauroyl peroxide) was added in about 5 ml of the liquid monomer solution in a test tube equipped with a polymer cap. The addition was done at room temperature, and the sample was shaked and then placed in a sample holder, which was in a tank of water kept at constant temperature of 65 °C with water circulation. Organic peroxide should be handled with care as they can decompose violently.

Within 2 minutes the color of the solution changed and became light brown.

After the crystallization trial as described in the table above, the test with the peroxide was repeated on the "mother Liquor" and on "melted crystals". A clear color difference could be seen, with the mother liquor giving a darker color.

### Example 4:

The crude obtained from White cast PMMA sheets was used in this test. The PMMA was depolymerized at 470 °C in the twin-screw extruder operated at 90 kg/h. The hot vapor were collected, washed with cold crude MMA in counter current mode, and condensed in a condenser formed of a tube and shell heat exchanger. A sample of a transparent clear solution was taken from the condenser.

The Peroxide test was repeated on the Crude MMA solution, and the color turned to a pink-brown within 2 minutes.

After the crystallization trial as described in the table above, the test with the peroxide was repeated on the "mother liquor" and on "melted crystals". A clear color difference could be seen with the mother liquor giving a darker color. This confirms that an impurity concentrated in the mother liquor.

### Example 5 and 6:

Crude MMA was obtained from depolymerization of a mix of Post-Consumer scraps of Cast and Extrusion sheets collected from companies cutting the sheets to dimensions and making various products from them. These are mixed colored cast and extrusion PMMA sheets with a ratio of about 80 wt % cast sheets and 20 wt % extrusion sheets, from various producers. The mix treated represents the average composition of what can be collected in Europe from this type of companies.

The PMMA was depolymerized at 470 °C in the twin-screw extruder operated at 90 kg/h. The hot vapor were collected, washed with cold crude MMA in counter current, and condensed in a tube and shell heat exchanger. A sample of a transparent clear solution was taken from the condenser.

The Peroxide test was repeated on the Crude MMA solution, and the color turned to a pink-brown within 2 minutes.

After the crystallization trial as described in the table above, the test with the peroxide was repeated on the "mother liquor" and on "melted crystals". A clear color difference could be seen;the mother liquor giving a darker color. This confirms that an impurity concentrated in the mother liquor.

The experiment was repeated about 30 minutes later, when a new sample was withdrawn from the condenser.

The same result was obtained.

### Example 7:

Crude MMA was obtained from injection PMMA scraps from car tail lights containing red, black and transparent particles was used in this test. The PMMA was depolymerized at 470 °C in the twin-screw extruder operated at 50 kg/h. The hot vapors were collected, washed with cold crude MMA in counter current, and condensed in condenser formed of a tube and shell heat exchanger. A sample of a transparent clear solution was taken from the condenser

The Peroxide test was repeated on the Crude MMA solution, and the color turned to a orange color within 2 minutes.

After the crystallization trial as described in the table above, the test with the peroxide was repeated on the "mother liquor" and of "melted crystals". A clear color difference could be seen; the mother liquor giving a darker color. This confirms that an impurity concentrated in the mother liquor.

### Example 8:

Crude MMA was obtained from equal amounts of a white cast PMMA used to make bath tubs, and contaminated with glass fibers and the adhesive that was used to stick the glass fibers on the PMMA to consolidate the bath tub, and, Impact resistant. The PMMA was depolymerized at 470 °C in the twin-screw extruder operated at a total feed rate of 50 kg/h. The hot vapor were collected, washed with cold crude MMA in counter current, and condensed in a condenser formed of a tube and shell heat exchanger. A sample of a transparent clear solution was taken from the condenser

The Peroxide test was repeated on the Crude MMA solution, and the color turned to an orange color within 2 minutes.

After the crystallization trial as described in the table above, the test with the peroxide was repeated on the "mother liquor" and of "melted crystals". A clear color difference could be seen; the mother liquor giving a darker color than the MMA from the second crystallization. This confirms that an impurity concentrated in the mother liquor.

## Claims

1. A method for the production of a monomer from a feed material containing a polymer of which the monomer is a constituent, the production method comprising the steps of:
- depolymerizing the feed material to obtain a monomer solution containing the monomer and impurities; and
- purifying the monomer solution thereby obtaining a purified monomer solution having a content of impurities lower than the content of impurities of the monomer solution,
wherein the purification step comprises at least one crystallization step each crystallization step comprising the steps of:
a) cooling of the monomer solution such that the monomer solution forms monomer crystals and a mother liquor,
b) separating the monomer crystals from the mother liquor, and
c) melting the monomer crystals thereby obtaining an enriched monomer solution.

2. The production method according to claim 1, wherein the purification step comprises subjecting the enriched monomer solution resulting from one crystallization step to another crystallization step.

3. The production method according to claim 1 or 2, wherein the purification step comprises subjecting the mother liquor resulting from one crystallization step to another crystallization step.

4. The production method according to anyone of the preceding claims, wherein the purification step comprises a water removal step.

5. The production method according to claim 4, wherein the water removal step comprises:
- the cooling of the monomer solution to a cooling temperature thereby transforming any water present in the monomer solution into ice without crystallizing the monomer, and
- separating the ice from the monomer solution,
whereby the at least one crystallization step is operated on the monomer solution after removal from the water.

6. The production method according to claim 5, wherein the cooling temperature is above the crystallization temperature of the monomer contained in the monomer solution.

7. The production method according to claim 6, wherein the difference between the cooling temperature and the melting point of the monomer contained in the liquid stream is higher than 4°C, preferably higher than 6°C and/or lower than 30°C, preferably lower than 20°C, mode preferably lower than 10°C.

8. The production method according to any one of claims 4 to 7, wherein during the water removal step, the monomer solution is cooled at least in part by heat exchange with the purified monomer solution resulting from crystallization.

9. The production method according to any one of claims 1 to 3, comprising adding at least one anti-icing additive in the monomer solution before implementing the at least one crystallization step such as to inhibit formation of ice during the implementation of the at least one crystallization step.

10. The production method according to claim 9, wherein the at least one anti-icing additive includes methanol.

11. The production method according to any one of the preceding claims, wherein the depolymerization step comprises the pyrolysis of the feed material in a pyrolysis reactor thereby generating a gas stream and the condensation of the gas stream in a condenser thereby obtaining the monomer solution.

12. The production method according to claim 11, wherein the depolymerization step comprises a separation step including removing solid and/or liquid impurities from the gas stream in a separating unit before condensing the gas stream.

13. The production method according to claim 12, comprising a step of cleaning the gas stream between the separating unit and the condenser using a gas cleaning unit fluidly connected in series between the separating unit and the condenser, the gas cleaning unit comprising a transfer column containing internals, the gas cleaning unit being fed with a fraction of the monomer solution exiting the condenser such that the gas stream flows upwardly in the transfer column and the monomer solution flows downwardly in the transfer column, heavy contaminants contained in the gas stream condense in liquid state on the internals and flow back down by gravity towards the bottom of the transfer column and preferably back to the separating unit.

14. The production method according to claim 12 or 13, wherein the separating unit comprises an auxiliary separator and a main separator fluidly connected in series.

15. The production method according to any one of claims 11 to 14, comprising a step of cleaning the monomer solution between the condenser and the purification unit by passing the monomer solution successively through an evaporator and a condenser thereby removing heavies from the monomer solution.

16. The production method according to any one of claims 11 to 15, comprising a washing step including the addition of water to the monomer solution upstream from the crystallization unit.

17. The production method according to any one of the preceding claims, wherein the polymer is polymethyl methacrylate (PMMA) and the monomer is methyl methacrylate (MMA).

18. The production method according to any one of the preceding claims, wherein the number of crystallization step is equal or lower than five, in particular equal or lower than three.

19. Installation for the production of a liquid monomer from a feed material containing a polymer of which the monomer is a constituent, the installation being configured for implementing a production method according to any one of the preceding claims.
